# NEUE EUROPÄISCHE PATENTSCHRIFT

(11) **EP 1 319 644 B2**
(45) Veröffentlichungstag und Bekanntmachung des Hinweises auf die Entscheidung über den Einspruch: **01.04.2009**
(45) Hinweis auf die Patenterteilung: 11.01.2006
(21) Anmeldenummer: 02025002.3
(22) Anmeldetag: 08.11.2002
(51) Int. Cl.: C07C 29/74, C07C 31/26

(54) **Verfahren zur Herstellung von direkt tablettierbaren Beta-Mannit**
Process for the preparation of directly compactible beta-mannitol
Procédé de préparation de beta-mannitol directement comprimable

(30) Priorität: 13.12.2001 DE 10161402
(43) Veröffentlichungstag der Anmeldung: 18.06.2003
(73) Patentinhaber: Merck Patent GmbH, 64293 Darmstadt (DE)
(72) Erfinder: Erdmann, Martin, 64521 Gross-Gerau (DE); Hamm, Walter, 64331 Weiterstadt (DE)

(56) Entgegenhaltungen:
- WO-A-00/76650
- US-A- 6 165 511
- PATENT ABSTRACTS OF JAPAN vol. 010, no. 258 (C-370), 4. September 1986 (1986-09-04) & JP 61 085330 A (FUJI KAGAKU KOGYO KK), 30. April 1986 (1986-04-30)
- BAUER H ET AL.: "Investigations on Polymorphism of Mannitol/Sorbitol Mixtures after Spray-drying using Differential Scanning Calorimetry, X-ray Diffraction and Near-Infrared Spectroscopy" PHARMAZEUTISCHE INDUSTRIE, Bd. 62, Nr. 3, 2000, Seiten 231-235, XP009008525

## Beschreibung

Die vorliegende Erfindung betrifft ein Verfahren zur Herstellung von direkt tablettierbarem Mannit mit einem Gehalt an β-Modifikation von mehr als 90 %.

Um das an sich nicht, bzw. nur mit besonderem Aufwand tablettierbare β-Mannit besser verarbeiten zu können ist es bekannt, Mannit gemeinsam mit Sorbit zu verwenden. Auch in der Veröffentlichung von Bauer, H. et al. [Pharm. Ind., Bd. 62, Nr. 3 (2000)] werden nur Untersuchungen zum Polymorphismus von sprühgetrockneten Mannit/Sorbit-Mischungen analysiert, zu deren Herstellung Lösungen mit einem Sorbit-Gehalt von 10 - 40 % bezogen auf den Feststoffgehalt eingesetzt werden. Es werden daher lediglich Zusammensetzungen mit einem hohen Sorbitgehalt untersucht, worin Mannit zuerst auszukristallisieren scheint und dabei die β-Modifikation ausbildet. Erfahrungsgemäß sind jedoch Mannitzusammensetzungen, in denen Sorbit in recht hohen Mengen enthalten sind, aufgrund des Sorbitgehalts im Gegensatz zu reinem Mannit recht gut tablettierbar.

Bei der Herstellung von Tabletten kann als Trägermaterial für einen Wirkstoff D-Mannit eingesetzt werden. Dazu wird üblicherweise das D-Mannit durch mehrere Verfahrensschritte mit entsprechenden Zwischenkontrollen in eine pulverförmige Form übergeführt, um es für die Tablettierpressen handhabbar zu machen und gleichzeitig eine Wirkstoffeinbindung zu ermöglichen.

Aus US 3,145,146 A ist ein Sprühtrocknungsverfahren bekannt, wodurch Mannit in Form feiner Partikel erhalten wird mit einem durchschnittlichen Durchmesser von 5 bis 150 µm. Eine Mannit-Lösung wird durch Versprühen in einen Heißgasstrom sprühgetrocknet. Die erhaltenen Partikel werden durch geeignete Maßnahmen abgetrennt. Aus JP 61085330, veröffentlich am 30. 04. 1986, ist darüber hinaus bekannt, D-Mannit aus einer wässrigen Lösung bei Temperaturen von 120 - 140 °C einer Sprühtrocknung zu unterziehen.

Zur Herstellung von pulverförmigem D-Mannit ist auch die Granulierung in einem Wirbelbett bekannt, worin der Prozeßluftstrom einen speziell geformten Anströmboden durchströmt, und dabei eine Wirbelschicht aus festem Startmaterial erzeugt. Die Sprühflüssigkeit gelangt durch ein Düsensystem feinverteilt in den Wirbelraum. Die wirbeinden Partikel werden benetzt, die Oberfläche angelöst, und die Partikel haften zusammen. Am Ende des Wirbelbettes wird kontinuierlich Feststoff entnommen. Gleichzeitig wird am Eingang eine geringere Menge Feststoff zugeführt, auf den Sprühflüssigkeit fein verteilt wird. Ein Filtersystem bewirkt, daß kein Staub das Wirbelbett verläßt und nur am Ausgang Granulatpartikel entnommen werden, die eine Mindestgröße aufweisen. Auch in einem solchen Wirbelbett bilden sich Festtsoffpartikel, die mehr oder weniger statistisch geformt sind. Entsprechende Anlagen werden durch verschiedene Hersteller vertrieben.

Üblicherweise schließt sich an die Herstellung eines pulverförmigen Mannits eine Verfahrensstufe an, durch die ein Pulver mit einer gleichförmigen Partikelgrößenverteilung erhalten wird. Diese Verfahrensstufe kann sowohl ein Mahlen als auch ein Sieben (Klassieren) des Pulvers einschließen. Im Fall der Verwendung von Mannit als Trägermaterial für pharmazeutische Wirkstoffe stellt für den Fachmann jeder zusätzliche Verfahrensschritt in der Herstellung eine mögliche Gefahr dar, unerwünschte Verunreinigungen in das Produkt einzutragen.

Aus der Literatur ist weiterhin bekannt, dass D-Mannit in polymorphen Kristallformen vorliegen kann, diese können die α-, β- und δ-Form sein. Die hier benutzten Definitionen und Charakterisierungen entsprechen der in : Walter Levy, L.; Acad. Sc. Paris, t. 267 Series C, 1779, (1968) gegebenen Klassifikation der polymorphen Formen durch Röntgenstrukturanalyse (X-ray diffraction pattern). Die β-Form ist die stabilste Form, obwohl Umwandlungen in die anderen Formen in Abhängigkeit von der Lagerzeit und den Bedingungen der Umgebung möglich sind. Für kommerzielle Anwendungen ist es daher wünschenswert, Mannit aufgrund seiner Stabilität direkt bei der Herstellung in der β-Form zu erhalten, da sich die Produkteigenschaften in diesem Fall am wenigsten durch Lagerung ändern.

Es ist weiterhin bekannt, dass es einerseits für die Tablettiereigenschaften des pulverförmigen D-Mannits von Bedeutung ist, in welcher polymorphen Form es vorliegt und andererseits in welcher Weise die Kornstruktur der einzelnen Partikel aufgebaut ist.

Durch WO 97/38960 A1 wird beschrieben, dass sich verbesserte Tablettiereigenschaften durch eine teilweise oder völlige Umwandlung des pulverförmigen D-Mannits aus der δ-Form in die β-Form ergeben. Die Umwandlung von der δ-Form in die β-Form wird hervorgerufen, durch gezielte Benetzung der Partikeloberflächen des Pulvers mit einem wasserlöslichen Lösungsmittel oder Wasser und durch anschließendes Trocknen. Der prozentuale Anteil an gebildetem β-Mannit ist abhängig von der eingesetzten Lösungsmittelmenge und der Dauer des Trocknungsvorgangs. Üblicherweise ist daher im Produkt ein Gemisch aus δ-und β-Form vorhanden.

Nachteilig ist an diesem Verfahren, dass sich die Umwandlung als zusätzlicher Verfahrensschritt an die eigentliche Pulverherstellung anschließt und die Trocknung mindestens 8 Stunden erfordert, wobei der Anlage kontinuierlich Wärmeenergie zugeführt werden muß.

Aufgabe der vorliegenden Erfindung ist es daher, ein Verfahren zur Verfügung zu stellen, das in einfacher Weise durchführbar ist und durch das ein direkt tablettierbares Mannit in einem Arbeitsschritt in der β-Modifikation herstellbar ist.

Die Lösung der Aufgabe erfolgt durch ein Verfahren zur Herstellung von direkt tablettierbarem Mannit mit einem Gehalt an β-Modifikation von mehr als 90 %, dadurch gekennzeichnet, dass
a) in einem ersten Schritt als Edukt eine wässrige 40 - 50%-ige D-Mannit-Lösung mit einer Temperatur im Bereich von 60 bis 95 °C mit Sprühgas, pulverförmigem β-Mannit und Heißgas zusammengeführt wird,
   wobei der Sprühdruck der verwendeten Zweistoffdüse im Bereich von 2 - 4 bar eingestellt und 1,5 bis 3 m³/(h kg Lösung) Heißgas mit einer Temperatur von 80 bis 110°C als Sprühgas der Zweistoffdüsezugeführtwird und die der Anlage zugeführte Luft (4) auf eine Temperatur im Bereich von 45- 120 °C vorgewärmt wird und in einer Menge im Bereich von 1000 - 2000 m³/m² pro Stunde zugeführt wird, wodurch sich die Ablufttemperatur im Bereich von 30-50 °C einstellt,
b) das entstehende pulverförmige Produkt in ein Wirbel- oder Fließbett fällt, aufgenommen, fluidisiert und weitertransportiert wird, und gegebenenfalls in einem oder mehreren Granulierungsschritt(en) mit weiterem flüssigen Medium besprüht, getrocknet und im Fließ- oder Wirbelbett weitertransportiert wird, und
c) durch Rückführung in einer Menge im Bereich von 0,2 - 2,0 kg Feststoff /(h/kg Lösung)
wobei durch Rückführung eines Pulvers mit Teilchengrößen kleiner 20 μm Mannitpulver mit einem Anteil an β-Fraktion von mehr als 90% erhalten wird.

Zur Herstellung der Mannit-Lösung wird D-Mannit mit einer Reinheit von >90 %, vorzugsweise von >95 % verwendet Vorzugsweise wird D-Mannit mit einer Reinheit von >98 % verwendet.

Überraschenderweise lässt sich das Gleichgewicht hin zur Bildung von β-Mannit verschieben, indem das als Staubanteil anfallende β-Mannits aus der Produktaustragszone des Prozessors in den Schritt a) der Sprühtrocknung rückgeführt wird. Besonders vorteilhaft gestaltet sich das Verfahren, indem β-Mannit mit einer durchschnittlichen Partikelgröße kleiner 20 µm, insbesondere mit durchschnittlicher Partikelgröße im Bereich von etwa 1 bis 20 µm, vorzugsweise im Bereich von 3 bis 15 µm, zurückgeführt wird.

Die Rückführung des "staubförmigen" β-Mannits, das aus der Pulverdosieranlage in der Leitung (9A) als pulverförmiges β-Mannit anfällt, erfolgt durch Steuerung der Drehzahl der Zellradschleuse 10A über den Ventilator (E) in die Sprühtrocknung (Schritt a).

Nach der Einstellung des Gleichgewichts ist es ohne weiteres möglich, pulverförmiges β-Mannit mit einer durchschnittlichen Partikelgröße kleiner als 75 µm rückzuführen, insbesondere aber auch pulverförmiges unvermahlenes β-Mannit.

Die besondere Ausgestaltung der verwendeten Anlage ermöglicht es, dass das zurückgeführte pulverförmige Material vor der Rückführung durch Vermahlen im Ventilator (E), der gleichzeitig als Förderorgan für die Pulverrückführung dient, zerkleinert wird.

Durch Regelung der Drehzahlen der Zellradschleusen 10A und 10B der verwendeten Anlage und durch Vermahlen des anfallenden grobkörnigen (Überkorn) Produkts im Ventilator (E) auf Partikelgrößen kleiner 75 µm vor der Rückführung in die Sprühtrocknung erfolgt die alleinige Bildung von β-Mannit.

Zur Durchführung des Verfahrens wird eine wässrige 40 - 50%-ge D-Mannit-Lösung als Edukt eingesetzt wird und mit einer Temperatur im Bereich von 60 bis 100 °C versprüht wird.

Sowohl als Sprühgas als auch als Träger- und Heizgas kann Luft oder ein Inertgas ausgewählt aus der Gruppe N₂ und CO₂ verwendet werden. Das Gas wird im erfindungsgemäßen Verfahren vorzugsweise im Kreislauf geführt und das im Kreislauf geführte Gas durch Filter von Partikeln befreit, im Kondensator getrocknet und erneut den Sprühdüsen zugeführt bzw. aufgeheizt und in das Fließbett eingeführt.

Vorzugsweise wird das im Kreis geführte das Gas mit Hilfe von Dynamikfiltern von Partikeln befreit.

In einer besonderen Ausführungsform des Verfahrens weisen die verwendeten flüssigen Medien an verschiedenen Stellen der Anlage unterschiedliche Zusammensetzungen auf.

Durch Variation der Verfahrensparameter, Sprühdruck, Sprühmenge, Mannitkonzentration, zurückgeführte Pulvermenge, Heißluftstrom und Temperatur der Heißluft lassen sich im erfindungsgemäßen Verfahren gezielt Partikelgrößen zwischen 50 bis 1000 µm herstellen.

Zu diesem Zweck wird erfindungsgemäß die der Anlage zugeführte Luft auf eine Temperatur im Bereich von 45 - 110 °C vorgewärmt und die zugeführte Zuluftmenge im Bereich von 1000 - 2000 m³/m² pro Stunde geregelt, wodurch sich die Ablufttemperatur im Bereich von 30 - 50 °C einstellt. Gleichzeitig wird der Sprühdruck der Zweistoffdüsen im Bereich von 2 - 4 bar eingestellt so dass etwa 1,5 bis 3 m³/(h kg Lösung) Heißgas der Zweistoffdüse zugeführt werden, wobei die Temperatur des Heißgases im Bereich von etwa 70 bis 110 °C eingestellt wird. Gute Verfahrensergebnisse werden erhalten, wenn die Pulverrückführung so geregelt wird, dass eine Rückführung in einer Menge im Bereich von 0,5 - 2,0 kg Feststoff/(h kg Lösung) erfolgt.

Eine besonders gleichförmige Bildung von pulerförmigem mit einem von β-Mannitgehalt >95% erfolgt durch Einstellung der Parameter, Sprühdruck, Flüssigkeitsmenge, Mannitkonzentration, zurückgeführte Pulvermenge, Heißluftstrom und Temperatur der Heißluft, wodurch die im Wirbel- oder Fließbett befindliche Pulvermenge auf eine Bettmenge im Bereich von 50 - 150 kg/m² Bett eingestellt wird.

Durch Versuche wurde ein Verfahren zur Herstellung von reinem β-(D)-Mannit gefunden, durch das direkt tablettierbares Mannit (DC-Mannit) mit geeigneter homogener Partikelgrößenverteilung herstellbar ist. Zur Durchführung des Verfahrens wird Mannit mit einer Reinheit von über 98% eingesetzt, wobei der Rest Sorbit und andere Restzucker sein können. Es wird eine wässrige Lösung mit einem Mannitgehalt von ca. 40 - 50 Gew.-% hergestellt. Diese Lösung wird mit einer Zulufttemperatur von ca. 60 - 95 °C in einer Sprühtrocknungsanlage versprüht und getrocknet.

Zur Durchführung des Verfahrens wird eine wie in DE 1 99 27 537 beschriebenen Anlage eingesetzt, die jedoch leicht verändert wurde. Mit der in dieser Patentanmeldung beschriebenen Anlage ist es an sich möglich, die Eigenschaften von sprühgetrockneten bzw. granulierten, pulverförmigen Produkten nach Wunsch hinsichtlich Komgröße, Komgrößenverteilung, Feuchte und Tablettierfähigkeit zu variieren. Die Veränderungen der Anlage ermöglichen jedoch eine zusätzliche Feinregulierung durch die Pulverrückführung.

Insbesondere erfolgt die Durchführung des Verfahrens in einer Sprühtrocknungsanlage, welche
a) eine Sprühtrocknungseinheit (B)
b) ein Fließbett (A)
c) eine oder mehrere zusätzliche Sprüh- oder Zerstäubungsdüsen für flüssige Medien (C)
e) einem Pulverdosiergerät (D) und
f) eine Pulverrückführung (9) mit Ventilator (E) aufweist, wobei die für die Pulverrückführung vorgesehenen Leitungen (9A) und (9B) mit Zellradschleusen (10A, 10B) versehen sind und das nicht in die Pulverdosierung eintretende Pulver (8) in eine staubförmige und eine grobkörnige Fraktion getrennt werden kann.

In der Sprühtrocknungseinheit (B) der erfindungsgemäß verwendeten Anlage werden flüssiges Medium (5), Sprühgas (6), pulverförmiges Material (9) und Heißgas (4) zusammengeführt.

Eine besondere Ausführungsform besteht darin, daß sich eine Sprühtrocknungseinheit (B) senkrecht über einem anschließenden Wirbelbett in einem Sprühturm befindet.

In einer speziellen Ausführungsform kann die Sprühtrocknungseinheit (B) der Anlage ein Sprühsystem enthalten, das aus einer mit Heißwasser beheizten Zweistoff-Sprühdüse mit koaxial angeordneter Pulverrückführung und Heißgasumströmung besteht.

In der verwendeten Anlage können im Fließbett auch örtlich variabel eine oder mehrere zusätzliche Sprüh- oder Zerstäubungsdüsen für flüssige Medien (C) angebracht sein. An das Fließbett schließt sich eine durch eine Schleusenklappe (F) abgetrennte Pulverdosieranlage (D) an, welche durch einen Produktüberlauf (8) gespeist wird. Eine Teilmenge des gebildeten Produkts kann über eine Flugförderung, in der ein Ventilator (E) als Förderorgan dient, gegebenenfalls nach Zerkleinerung (9A,10A) oder unzerkleinert (9B, 10B) in die Sprühtrocknungseinheit (B) zurückgeführt werden. Der als Förderorgan wirkende Ventilator (E) kann gleichzeitig als Zerkleinerungseinheit des rückgeführten Pulvers dienen.

Die Durchführung des Verfahrens zur Herstellung von sprühgetrocknetem pulverförmigen β-(D)-Mannit erfolgt , indem
a) in einem ersten Schritt ein flüssiges Medium, Sprühgas, pulverförmiges Material und Heißluft zusammengeführt werden,
b) das bestehende pulverförmige Produkt in ein Fließbett fällt, aufgenommen, fluidisiert und weitertransportiert wird und gegebenenfalls
c) in einem oder mehreren Granulierungsschritt(en) mit weiterem flüssigen Medium besprüht, getrocknet
d) und im Fließbett in Richtung der Pulverdosieranlage gefördert wird, von welcher aus
e) eine Teilmenge ungemahlen und/oder gemahlen als pulverförmiges Material in den Prozeß zurückgeführt wird.

Bei dem flüssigen Medium handelt es sich vorzugsweise um eine Lösung. Es kann aber auch eine wässrige Suspension von bereits gebildetem β-(D)-Mannit sein.

Eine besondere Variante des Verfahrens besteht darin, daß das zurückgeführte pulverförmige Material vor der Rückführung zerkleinert werden kann.

Als Sprüh-, Träger- und Heizgas kann Luft oder ein Inertgas ausgewählt aus der Gruppe N₂ und CO₂ verwendet werden. Das Gas kann erfindungsgemäß im Kreislauf geführt werden, wobei es durch Filter oder speziell mit Hilfe von Dynamikfiltern von Partikeln befreit wird, im Kondensator getrocknet und erneut den Sprühdüsen zugeführt bzw. aufgeheizt und in das Fließbett eingeführt wird.

Zur Durchführung des Verfahrens wird zu Beginn die Anlage mit Pulverförmigem Startermaterial über den Einfüllstutzen (3) beschickt. Über die Kammern (1) wird im Sprühtrocknungsraum ein Luftstrom erzeugt. Das eingeführte Startermaterial wird durch diesen Luftstrom fluidisiert und bewegt sich in die Richtung der Austragsklappen (F). Der Pulverstrom erhält diese Bewegungsrichtung bei Erzeugung des Luftstroms durch eine entsprechende Perforation des Conidurbodens. Das fluidisierte Produkt läßt sich durch einfaches Öffnen der Schleusenklappen (F) austragen. An dieser Stelle der Anlage sind Vorrichtungen geschaffen, die es ermöglichen, entweder das Produkt in eine Pulverdosieranlage oder über eine Flugförderung zur Sprühtrocknungseinheit zurück zu führen. Am Austrag über die Pulverdosieranlage befindet sich ein Überlauf (8) für das Ferigprodukt. Der Ventilator (E) der Sprühtrocknungseinheit dient sowohl als Fördermittel für das Produkt als auch als Zerkleinerungseinheit für zurückzuführendes Pulvermaterial. Zurückgeführtes Pulvermaterial der Rückführungsleitung (9A, 9B) wird durch die besondere Ausgestaltung der Sprühtrocknungsdüse mit den entsprechenden Medien Flüssigkeit (5), Sprühluft (6) und Heißluft (4) zusammengeführt. Das entsprechende Pulver bzw. Granulat wird vom Fließbett aufgenommen und, wie bereits oben beschrieben, weitertransportiert. Beim Passieren der Granulationsdüsen (C) kann weiteres Medium, welches eine andere Zusammensetzung haben kann als das in die Sprühdüse mit Pulverrückführung eingebrachte, auf die gebildeten Partikel gesprüht. Auf diese Weise kann ein weiteres Granulieren und eine erneute Einstellung der Korngrößenverteilung erfolgen. Das Produkt aus den Kammern (1) wird durch Luft, welche über die Conidurböden eingeschleust wird, auf die gewünschte Endfeuchte getrocknet. Durch in die Anlage integrierte Dynamikfilter (G) wird ein Austrag von Pulverpartikeln in die Umgebung verhindert.

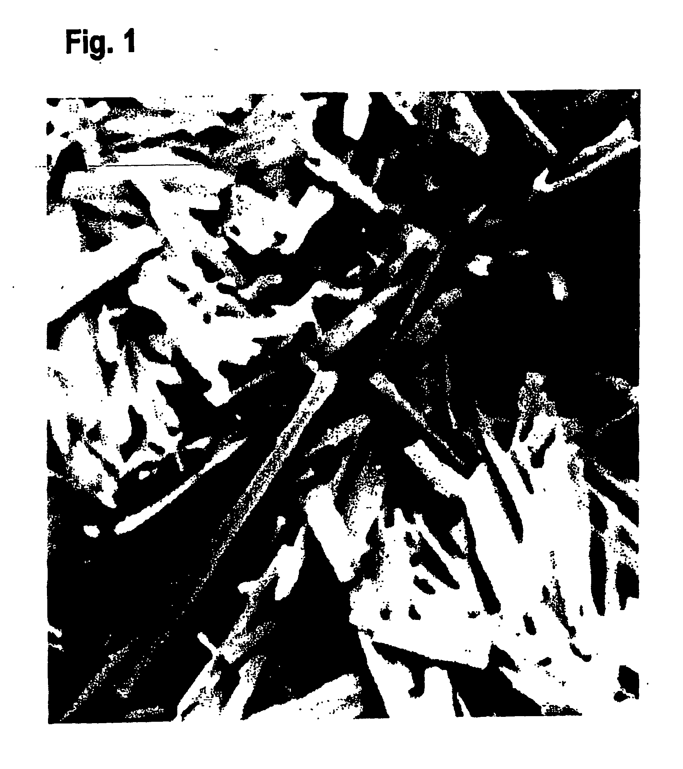

Statt der Granulationsdüsen (C), wie in Abbildung 1 dargestellt, können an der entsprechenden Stelle der Anlage ein oder mehrere Sprühdüsen oder Sprühtrocknungsdüsen oder auch nur eine, zwei oder mehr als drei Granulationsdüsen angebracht sein. Diese zusätzlichen Düsen können sich direkt am Anfang des Fließbettes oder weiter nach hinten verschoben befinden. Die Wahl des Ortes, an dem das ursprünglich gebildete Pulvermaterial erneut ein- oder mehrmal(s) besprüht wird, ist u.a. auch davon abhängig, welche Restfeuchte das gewünschte Produkt haben soll. Es versteht sich von selbst, daß ein Produkt mit besonders niedriger Restfeuchte nach dem letzten Besprühen eine längere Verweilzeit im Wirbelbett erforderlich macht als eines mit höherer.

Je nach Wunsch können durch die verschiedenen Düsen unterschiedliche Zusammensetzungen auf die bereits gebildeten Partikeloberflächen aufgebracht werden, so daß Partikel mit einem schichtförmigen Aufbau erhalten werden können. Es kann aber auch dazu dienen, eine gleichmäßigere Kornverteilung zu erzielen.

Weiterhin kann die Anlage nicht nur mit Luft als Trägermedium betrieben werden. Es ist auch möglich, die gesamte Anlage im Kreislauf mit einem Inertgas, wie z.B. Stickstoff oder Kohlendioxid, zu fahren.

Die Anlage ist so ausgestaltet, daß die Parameter Flüssigkeitsmenge, Sprühdruck, rückgeführte Pulvermenge, Heißgasmenge, Heißgastemperatur, Warmluftmenge, Warmlufttemperatur usw. individuell regelbar sind. Daher lassen sich die zurückgeführte Pulvermenge, die eingespeiste Flüssigkeitsmenge und den Sprühdruck gezielt einstellen je nach gewünschten Eigenschaften hinsichtlich der Feuchte, der Korngröße und der Korngrößenverteilung des Endproduktes. Je nach Wunsch können in der beschriebenen Anlage pulverförmige Produkte hergestellt werden mit Partikelgrößen zwischen 50 und 1000µm. Je nach Fahrweise und gewählten Verfahrensparametern können die Partikel einen schichtförmigen (Zwiebelstruktur) Aufbau oder je nach eine Agglomeratstruktur aufweisen.

Besonders gesteuert werden kann die Bildung der Partikel durch eine in die Anlage integrierte Sprühdüse, welche geeignet ist, sprühgetrocknete Granulate herzustellen. Bei dieser Sprühdüse handelt es sich um ein Sprühsystem (B), das aus einer mit Heißwasser beheizbaren Zweistoff-Sprühdüse [(1), (2), (3)] besteht, welche wiederum mit einer um die Zweistoff-Sprühdüse angeordneten Pulverrückführung (4) und einer Heißgasumströmung (5) ausgestattet ist. Im Spezialfall kann die Pulverrückführung (4) koaxial um die Zweistoff-Sprühdüse angeordnet sein.

Vorteil dieses Sprühsystems ist, daß rückgeführtes Pulver unmittelbar am Austritt der Zweistoff-Sprühdüse mit den über die Zerstäubungsluft erzeugten Flüssigkeitströpfchen in Kontakt kommt. Damit die Pulverpartikel nicht verkleben und die Oberflächenfeuchte abgeführt werden kann, ist das Sprüh- und Pulverteil in einen Heißgasstrom eingeschlossen. Die Nachtrocknung auf die gewünschte Restfeuchte findet im Fließbett statt.

Insbesondere auch durch den Einbau dieses Sprühtrocknungssystems ist es möglich, Partikelgrößen gezielt herzustellen.

Ein besonderer Vorteil dieses Sprühtrocknungsverfahrens besteht daher darin, dass sich in einer einzigen Anlage ohne weitere Verfahrensschritte zur Nachbehandlung des Produkts in Abhängigkeit von den eingestellten Verfahrensparametern und von den verwendeten zu versprühenden flüssigen Medien pulverförmiges β-(D)-Mannit mit sehr unterschiedlichen Eigenschaften hinsichtlich Feuchte, der Korngröße und der Korngrößenverteilung des herstellen lässt.

Um besonders gute DC-Eigenschaften (DC = direkt tablettierbar) des sprühgetrockneten Stoffes zu erhalten, hier Mannit, ist es vorteilhaft, in der Sprühtrocknung gebildete Einzelpartikel zu agglomerieren. Zu diesem Zweck befindet sich senkrecht über dem Wirbelbett ein Sprühturm über der erfindungsgemäßen Sprühtrocknungseinheit (B).

Die heiße wäßrige Mannitlösung wird über eine oder mehrere Zweistoffdüse(n) (5) (6), die mit Heißwasser (7) beheizt wird (werden), versprüht. Der erzeugte Sprühstrahl wird von einer um diese Düse angeordneten Mannit-Pulverrückführung aus (9) und Heißgas (4) umströmt. Der Feststoff kristallisiert in dem Sprühstrahl aus, bildet Agglomerate und wird vom Fließbett aufgefangen. Das Fließbett wird von heißer Luft aus den Lufteinführungskammern (1) durchströmt und fluidisiert. Der Boden des Fließbettes ist ein Conidurboden, der für den gezielten Transport des Feststoffes in Richtung Austrag sorgt, und auch eine definierte Verweilzeit des Feststoffes im Fließbett ergibt. Durch die Betthöhe, Sprüh- und Rückführmenge kann die Verweilzeit des Produkts im Prozessor gesteuert werden. Der Feststoff wird über mehrere in Reihe geschalteten Lufteinführungskammern (1) transportiert und auf eine Restfeuchte <0,3% getrocknet. Der Trocknungsvorgang erfolgt über die Länge des Fließbettes in einem bestimmten Temperaturprofil, um eine Überhitzung des Produktes zu vermeiden.

Die mit Wasser beladene und staubhaltige Wirbelluft wird über Dynamikfilter (G) gereinigt und durch die Abluftkammern (2) abgeführt. Die Dynamikfilter werden mit Druckluftstößen regelmäßig abgereinigt. Der abgereinigte Staub bindet die Sprühnebel aus der Sprühzone und verhindert ein Festsetzen und Anbacken von Feststoff an den Wänden.

Der getrocknete Feststoff fällt über Doppelpendelklappen (F) oder andere Austragssysteme in ein Dosiersystem der Rückführung (D). Das ausgetragene Produkt kann optional über ein Klassiersystem weiter aufgearbeitet werden. Das entstehende Überkorn (- und Unterkorn) kann über die Pulverrückführung (9) im Ventilator (E) gemahlen und zusammen mit dem Unterkorn, also mit staubförmigem Mannitpulver mit Korngrößen kleiner 75 µm, insbesondere kleiner 40 µm, in den Sprühtrockner rückgeführt werden.

Ein Teilstrom wird am Austrag als fertiges Produkt (8) ausgeschleust. Das Produkt kann über ein Sieb klassiert werden, wobei das Überkorn oder Remanenz, d. h. der grobkörnige Pulveranteil über die Saugseite des Mahlventilators (9A) rückgeführt, gemahlen und wieder in den Prozeß zurückgegeben werden kann. Dadurch werden u. a. die Produktverluste minimiert.

Der Ventilator (E) der Sprühtrocknungseinheit dient sowohl als Fördermittel für zurückzuführendes Produkt (Feststoffaufgabe druckseitig (9B)) als auch als Zerkleinerungseinheit für rückgeführtes Pulvermaterial (Feststoffaufgabe saugseitig (9A)). Die beiden Feststoffteilströme werden z.B. über die Drehzahl der Zellradschleusen (10A, 10B) gesteuert. Zurückgeführtes Pulvermaterial der Rückführleitungen (9) wird, wie oben bereits beschrieben, durch die besondere Ausgestaltung der Sprühtrocknungsdüse mit den entsprechenden Medien Flüssigkeit (Mannitlösung) (5), Sprühluft (6) und Heißluft (4) zusammengeführt.

Aus der Produktaustragszone des Prozessors wird die Zuluft dem Ventilator (E) zugeführt. Auf diese Art und Weise wird gleichzeitig aus dem Produkt der Feinstaub (<15µm) entfernt (pneumatische Klassierung). Gleichzeitig hat die Entfernung dieses Feinstaubs den Effekt, dass bei Verwendung dieses von Feinstaub befreiten Produkts höhere Tablettenhärten erreicht werden können.

Bei dem Teilstrom 9B besteht nach der Zellradschleuse 10B die Option, das Überkorn (Remanenz) aus der Rückführung abzusieben, um das Verfahren besser steuern zu können. Dieses Überkorn (Remanenz) kann saugseitig dem Mahlventilator (E) oder einer anderen Zerkleinerungsmaschine zugegeben, gemahlen und wieder dem Prozeß zugeführt werden.

Wie oben schon angedeutet, lässt sich die Qualität der Agglomerate und damit des Produktes über die Anlagenparameter wie Konzentration, Sprühdruck, Temperatur, Sprühmenge, Pulverrückführungsmenge, Hauptluftmenge, Staubabsaugung, Betthöhe usw. steuern. Durch eine Reduzierung der Höhe der Sprühdüse [(B)→(C)] über dem Fließbett ist es möglich, die Kornstruktur von einem Agglomerat (Beerenstruktur) in ein Granulat (Zwiebelstruktur) zu überführen. Bei der tiefstmöglichen Anordnung der Düsen (Granulationsdüsen (C)) kann die Pulverrückführung (9) über den Einfüllstutzen (3) erfolgen. Um kontinuierlich ein direkt tablettierbares Produkt zu erhalten, müssen sowohl die Kornstrukturen, als auch Modifikation, Korngrößenverteilung, Wassergehalt, Dichte, usw. überwacht werden. Es wurde gefunden, dass das beste tablettierbare Produkt erhalten wird, wenn Mannit in einer feinen Nadelstruktur auskristallisiert.

Verunreinigungen durch andere Mannit-Modifikationen im Mannitpulver verschlechtern die Tablettiereigenschaften. Insbesondere wurde gefunden, dass vor allem zunehmende Anteile an α-Mannit sich negativ auf die Tablettierbarkeit, die erzielbaren Tablettenhärten und auf die Oberflächenbeschaffenheit der Tabletten auswirken. In Produkten, die nach dem erfindungsgemäßen Verfahren hergestellt worden sind, ist an sich kein α-Mannit nachweisbar, insbesondere im Mannit, welches einen Anteil an β-Modifikation von mehr als 95 Gew.-%, insbesondere von mehr als 98 Gew.-% aufweist.

In Abb.1 ist eine REM-Aufnahme eines Produkts mit einem Anteil an β-Modifikation von mehr als 98% in einer 500-fachen Vergrößerung wiedergegeben.

Durch Versuche wurde gefunden, dass es notwendig ist, die eingestellten Parameter des Sprühtrocknungsprozesses einzuhalten und zu überwachen, um ein gleichbleibend, gut tablettierbares, reines β-Mannit zu erhalten.

Erfindungsgemäß wird vorzugsweise als Edukt D-Mannit mit einer Reinheit >90%, insbesondere bevorzugt mit einer Reinheit von >95% und ganz besonders bevorzugt mit einer Reinheit >98%, eingesetzt. Dieses Edukt wird als wässrige 40 - 50%-ge Lösung eingesetzt und in die Anlage mit einer Temperatur im Bereich von 60 bis 95 °C versprüht. Vorzugsweise wird die Lösung vor dem Versprühen auf eine Temperatur im Bereich von 70 bis 85 °C, insbesondere von 75 bis 80°C, erwärmt.

Erfindungsgemäß können an verschiedenen Stellen der Anlage Lösungen mit unterschiedlichen Mannitkonzentrationen eingesetzt werden. So hat es sich als sinnvoll erwiesen, Sprühdüsen oberhalb des Fließbetts in Richtung des Produktaustrags mit Lösungen mit höheren Mannitkonzentrationen zu beschicken als Sprühdüsen, welche sich am Anfang des Fließbetts befinden. Es kann daher zum Ende des Fließbetts hin eine Lösung mit einer Mannitkonzentration von etwa 60 Gew.-% bezogen auf die Gesamtlösung eingesetzt werden, wohingegen die Zweistoffdüse mit Pulverrückführung bevorzugt mit einer etwa 40 - 50%-gen wässrigen Lösung betrieben wird. Auf diese Weise können die Produkteigenschaften nochmals im gewünschten Sinn beeinflusst werden, wobei bei dieser Fahrweise die Anlagenparameter genau zu beachten sind.

Durch Variation der Parameter, Sprühdruck, Flüssigkeitsmenge, zurückgeführte Pulvermenge, Heißluftstrom und Temperatur der Heißluft lassen sich gezielt Partikelgrößen zwischen 50 bis 1000 µm einstellen.

Weiterhin wurde gefunden, dass die Parameter der erfindungsgemäß verwendeten Anlage folgendermaßen eingestellt werden müssen, um ein gleichbleibendes Produkt zu erhalten:

Der Sprühdruck der Zweistoffdüsen ist im Bereich von 2 - 4 bar einzustellen, vorzugsweise wird im Bereich von 2,5 bis 3,5 bar gearbeitet. Die der Zweistoffdüse zugeführte Menge Heißgas ist so zu regulieren, dass etwa 1,5 bis 3 m³/(h kg Lösung) mit einer Temperatur von etwa 80 bis 110 °C gefördert werden. Es wurde gefunden, dass bei höherer Heissgaszufuhr eine bessere Produktqualität erhalten wird, wenn mit niedrigerer Temperatur gearbeitet wird.
Die Pulverrückführung ist erfindungsgemäß so einzustellen dass eine Feststoffrückführung im Bereich von 0,2 - 2,0 kg Feststoff/(h kg Lösung) erfolgt. Vorzugsweise wird im Bereich von 0,5 bis 1,5 kg Feststoff/(h kg Lösung) gearbeitet. Besonders günstig gestaltet sich der Prozess, wenn die Feststoffrückführung im Bereich von 0,5 bis 1,0 kg Feststoff/(h kg Lösung) liegt.
Zur Durchführung des Verfahrens muss in die Anlage vorgewärmte Luft eingespeist werden. Gute Ergebnisse werden erzielt, wenn die der Anlage zugeführte Luft auf eine Temperatur im Bereich von 45 - 120 °C vorgewärmt wird. Günstig ist es für den erfindungsgemäßen Prozess, wenn die Zuluft eine Temperatur im Bereich von 65 bis 110 °C aufweist. Besonders vorteilhaft für die Bildung eines β-Mannitpulvers mit guten Tablettiereigenschaften ist es, wenn die Temperatur der eingespeisten Zuluft im Bereich von 70 bis 100 °C liegt.
Die zugeführte Zuluftmenge ist erfindungsgemäß so zu regeln, dass 1000 - 2000 m³/m² pro Stunde, insbesondere 1200 bis 1700 m³/m² pro Stunde, in die Anlage eingespeist werden.

In Verbindung mit den übrigen eingestellten Parametern liegen günstige Verfahrensbedingungen vor, wenn der Luftstrom in der Anlage so geführt wird, dass sich die Ablufttemperatur im Bereich von 30 -50 °C einstellt.

Weiterhin hat es sich als günstig herausgestellt, die Verfahrensbedingungen so zu regulieren, dass sich die im Wirbel- oder Fließbett befindliche Pulvermenge auf eine Bettmenge von 50 - 150 kg/m² Bett einstellt. Besonders günstig ist es, wenn die Bettmenge im Bereich von 80 - 120 kg/m² Bett liegt.

Es wurde auch gefunden, dass sich der Prozess insbesondere durch gezielte Rückführung eines Pulvers mit ausgewählter Partikelgröße steuern lässt.

Wie aus dem Anlagenschema zu erkennen ist, kann eine Pulverrückführung sowohl durch Pulverentnahme aus dem Fließbett als auch durch Rückführung einer sehr feinteiligen Pulverfraktion, welche bei der Konfektionierung, d. h. Homogenisierung der Partikelgrösse durch Siebung und Abfüllung des hergestellten Produkts anfällt, erfolgen.

Es ist auch möglich, vor der Rückführung vorab Pulver mit größeren Partikelquerschnitten im Ventilator (E) der Sprühtrocknungseinheit zu zerkleinern. Wie oben bereits angedeutet, kann der Pulverstrom durch die Einstellung der Drehzahl der Zellradschleusen (10A, 10B) gesteuert werden. Um vor der Rückführung rückzuführendes Pulver auf die gewünschte Partikelgröße vorab zu vermahlen, ist dementsprechend die Drehzahl der Zellradschleuse 10A (B) so einzustellen, dass eine Rückführung über den Ventilator unter Vermahlung erfolgt.

Versuche haben gezeigt, dass das Gleichgewicht zur Bildung von β-Mannit hin verschoben werden kann, wenn die durchschnittliche Partikelgröße des rückgeführten, im Ventilator (E) gemahlenen Pulvers kleiner als 75 µm ist. Insbesondere erfolgt bevorzugt die Bildung von β-Mannit, wenn die durchschnittliche Teilchengröße des rückgeführten Pulvers geringer als 40 µm ist. Überraschend wurde gefunden, dass durch Rückführung eines Pulvers mit Teilchengrößen kleiner 20 µm Mannitpulver mit einem Anteil an β-Fraktion von mehr als 90 % erhalten wird. Besonders überraschend wurde gefunden, dass insbesondere durch die Rückführung des sogenannten Staubanteils, der in der Produktaustragszone des Prozessors anfällt und üblicherweise aus dem Produkt entfernt wird, zu einem gleichmäßigen Produkt mit einem besonders hohen Anteil an β-Fraktion führt. Die durchschnittliche Partikelgröße des Staubanteils liegt im Bereich von etwa 1 bis 20 µm, insbesondere im Bereich von 3 bis 15 µm. Außerdem wurde gefunden, dass der Staub aus der Rückführung zu einer stabilen Fahrweise in der Sprühzone des Prozessors führt.

Da durch Vermahlen im Ventilator (E) diese Partikelgrößen sich nur mit besonderem Aufwand erzielen lassen, wird insbesondere zu Beginn der Durchführung des Verfahrens bevorzugt der "staubförmige" Produktanteil aus der Pulverdosieranlage, der in der Anlage in der Leitung (9A) anfällt, durch Steuerung der Drehzahl der Zellradschleuse 10A durch Vermahlen in die Sprühtrocknung rückgeführt. Durch gleichzeitige Drosselung der Drehzahl der Zellradschleuse 10 B wird eine Rückführung grobkörnigen Mannitanteils reduziert.

Überraschenderweise wurde gefunden, dass der Prozess nach Einstellung des Gleichgewichts in Richtung zur Bildung von β-Mannit in einer Reinheit von mehr als 95 % der Prozess stabil weitergeführt werden kann, wenn ebenfalls das im Ventilator auf eine Partikelgröße von kleiner 75 µm vermahlene Pulver rückgeführt wird.

Auf diese Weise ist es überraschenderweise möglich, den Sprühtrocknungsprozess durch alleinige Rückführung des anfallenden "Staubanteils" durch geeignete Regelung der Drehzahlen der Zellradschleusen 10A und 10B zu Beginn so einzustellen, dass nur die Bildung von β-Mannit erfolgt. Anschließend kann auch der anfallende grobkörnigere Anteil (das sogenannte Überkorn) des gebildeten Mannitpulvers wieder in den Prozess rückgeführt werden, ohne eine Verschiebung des Gleichgewichts zu riskieren. Dieses hat den Vorteil, dass ein Anhaften der besonders feinteiligen, Sprühnebels an den Wänden der Anlage im Dauerbetrieb vermieden werden kann, und Störungen des Prozessablaufs unterbunden werden können.

Durch geeignete Wahl der Verfahrensparameter lässt sich ein Produkt mit einem Gehalt an β-Modifikation von mehr als 90 % herstellen. Durch ständige Kontrolle der hergestellten Produktqualität lässt sich der Anteil ohne weiteres auf einen Gehalt an β-Modifikation auf über 95 % steigern.

Insbesondere bei optimaler Einstellung der oben beschriebenen Anlagenparameter und Kotrolle der übrigen Verfahrensparameter wird im erfindungsgemäßen Verfahren ein Mannit als Produkt mit folgenden Eigenschaften erhalten:
- direkt tablettierbares Mannit
- Reinheit der β-Modifikation > 98%
- Schüttdichte 350 - 500 g/l
- Restfeuchte < 0,3%
- Partikelverteilung: x₅₀ bei 200 µm: <10% <53µm + <15% >500µm
   x₅₀ bei 300µm: <10% <100µm + <10% >850µm
   x₅₀ bei 450µm: < 5% <100µm + <10% >850µm

Da die unterschiedlichen Modifikationen des Mannits sehr ähnlich sind, lassen sie sich nicht aufgrund ihrer in der Analytik üblicherweise gemessenen Schmelztemperaturen in der DSC unterscheiden. Die Identifizierung ist ausschließlich z.B. mittels X-Ray oder NIRS möglich.

Aufgrund der mit dem hergestellten Produkt erzielten Tablettenhärten lassen sich jedoch deutliche Unterschiede zu handelsüblichen Produkten feststellen. Im Vergleich zu einem handelsüblichen Produkt, welches einen relativ hohen Anteil an β-Modifikation im pulverförmigen Mannit aufweist, werden mit dem erfindungsgemäß hergestellten β-Mannit Tabletten mit etwa 45 bis 70 % höheren Tablettenhärten erhalten.

Zum besseren Verständnis und zur Verdeutlichung der Erfindung werden im folgenden ein allgemeines Fließschema (Fig. 1) der beschriebenen Sprühtrocknungsanlage und Beispiele gegeben, die im Rahmen des Schutzbereichs der vorliegenden Erfindung liegen, nicht jedoch geeignet sind, die Erfindung auf diese Beispiele zu beschränken.

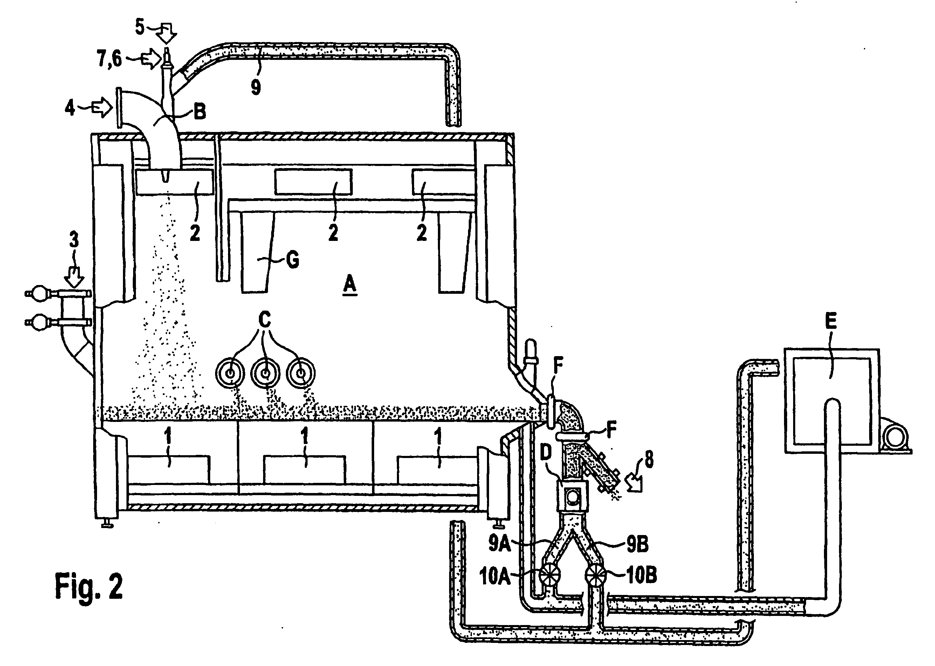

Abb. 2 zeigt ein verallgemeinertes Fließschema einer möglichen Ausführungsform einer zur Durchführung des Verfahrens eingesetzten Sprühtrocknungsanlage, in der die gegebenen Ziffern und Buchstaben die folgenden Bedeutungen haben:
- 1: Lufteinführungskammern
- 2: Heizvorrichtungen
- 3: Einfüllstutzen
- 4: Heißluftzufuhr
- 5: Flüssigkeitszufuhr
- 6: Sprühluft
- 7: Heizmedium
- 8: Produkt
- 9: Pulver
(9A feinteiliges Pulver (Staub), 9B grobkörniges Pulver)
- 10: Zellradschleuse (10A und 10B) zur Regelung der Pulverrückführung
- A: Fließbettapparat
- B: Sprühtrocknungseinheit
- C: Granulationsdüsen
- D: Pulverdosiergerät
- E: Ventilator zur Pulverrückführung
- F: Schleusenklappen
- G: Dynamikfilter

Anhand der in der Beschreibung genannten und den in dem Fließschema gegebenen Komponenten ist es dem Fachmann ohne weiteres möglich, zur Durchführung des Verfahrens durch Auswahl im Handel erhältlicher Einzelkomponenten eine entsprechende Anlage zu erstellen. Es versteht sich für den auf dem Fachgebiet tätigen Fachmann von selbst, dass zum Betrieb der Anlage sowohl zusätzliche elektrische als auch mechanische Regelungseinheiten eingebaut werden müssen, um die Parameter im erfindungsgemäßen Verfahren, wie beschrieben, regeln und variieren zu können.

Die folgenden Beispiele zur Herstellung von verschiedenen DC β-Mannit - Qualitäten dienen zur näheren Erläuterung der vorliegenden Erfindung.

### Beispiel 1

### Herstellung eines DC β-Mannits mit einem mittleren Kom X₅₀ = 200 µm

Die Sprühtrocknungsanlage wird zur Vorbereitung mit ca. 70 kg/m² β-Mannit als Bett befüllt. (Dieses vorgelegte Bett sollte möglichst die gewünschten Produkteigenschaften haben. Falls das zur Verfügung stehende Bettmaterial andere Eigenschaften besitzen sollte, so muss man die Anlage schonend anfahren, bis sich das Gleichgewicht in die gewünschte Richtung verschoben hat.)

Als Wirbel- und Zuluft wird die Anlage mit 1200 m³/m² h bei einer Temperatur von ca. 70° C betrieben. (Vor dem Anfahren der Anlage ist darauf zu achten, dass ausreichend Staub in der Anlage vorhanden ist. Es kann über die Pulverdosierung (D), der saugseitigen Rückführung (9A) und Dosierung (10 A) über den / mit dem Ventilator (E) Staub erzeugt und in die Anlage geblasen werden.) Ist genug Staub in der Anlage, wird die Dosierung (10 A) der Rückführung reduziert werden, und es wird mit dem Versprühen von Mannitlösung begonnen. Die versprühte Lösung hat eine Konzentration von ca. 40% und eine Temperatur von ca. 75° C. Bei einem Sprühdruck von ca. 3 bar (Sprühmedium ist Luft.) werden ca. 45 kg/m² h Lösung in der Anlage versprüht. Über die Pulverdosierung (D) werden ca. 0,5 kg Feststoff/(h kg Lösung) über die Rückführung (9, 10) in die Sprühzone rückgeführt. Die Zellräder (10 A, 10 B) werden so geregelt, dass immer eine ausreichende Menge Produkt (9A, 10A) im Ventilator (E) gemahlen und mit dem ungemahlenen Produkt (9B,10 B) wieder in die Anlage gefördert wird.

In der Anlage bildet sich durch die Verdunstung des Wassers ein Gleichgewicht mit einer Bettemperatur von ca. 35-40° C. Die Ablufttemperatur beträgt etwa 30-33° C. (Es ist darauf zu achten, dass die Abluft möglichst gesättigt ist (ca. 80 90% rel. Feuchte). Dies ist von Vorteil für den Wirkungsgrad des Prozesses als auch für den Kristallisationsvorgang des Mannits.) Auf diese Art und Weise erhält man die beste Kristallstruktur und die reinste β-Modifikation des Mannits. Da der Ventilator (E) seine Zuluft aus der Produktaustragszone vor den Schleusenklappen (F) der Anlage (A) holt, und somit das ausgetragene Produkt durch die pneumatische Klassierung staubfrei ist, erhält man am Produktaustrag (8) das β-Mannit mit hervorragenden Eigenschaften für die direkte Tablettierung. Um DC β-Mannit mit der gewünschten Korngrößenverteilung zu erhalten, kann es nach der Austragsschleuse (F), d. h. vor dem Produktaustrag (8) und der Pulverdosierung (D), gesiebt werden. Es ist für den Prozess von Vorteil, auch das Überkorn aus dem rückzuführenden Produkt (9 B,10 B) auszusieben, da dieses sich sonst in der Sprühzone weiter anreichert und im Fließbett Probleme bereiten kann. Das ausgesiebte Unter- und Überkorn kann saugseitig dem Ventilator (E) zugeführt, gemahlen werden, und zusammen mit den anderen rückgeführten Feststoffteilströmen (9A, 10 A, 9B, 10 B) wieder dem Prozess zugeführt werden. Auf diese Art und Weise werden die Produktverluste minimiert, und der Prozess läuft durch die zusätzliche Staubrückführung (gemahlenes Produkt) stabiler.

### Beispiel 2

### Herstellung eines DC β-Mannits mit einem mittleren Korn X₅₀ = 300 µm

Wie in Beispiel 1 beschrieben wird die Sprühtrocknungsanlage zur Vorbereitung mit ca. 100 kg/m² β-Mannit als Bett befüllt und angefahren.

Als Wirbel- und Zuluft wird die Anlage mit 1500 m³/m² h bei einer Temperatur von ca. 85° C betrieben. Die zu versprühende Mannit-Lösung hat eine Konzentration von ca. 45 Gew.-% bei einer Temperatur von ca. 80° C. Bei einem Sprühdruck von ca. 3 bar (Sprühmedium ist Luft) wird eine Lösungsmenge von ca. 65 kg/m² h in der Anlage versprüht. Die Zellräder (10 A, 10 B) werden so geregelt, dass immer eine ausreichende Menge Produkt (9A, 10A) im Ventilator (E) gemahlen und mit dem ungemahlenen Produkt (9B, 10 B) wieder in die Anlage gefördert wird. In der Anlage bildet sich durch die Verdunstung des Wassers ein Gleichgewicht mit einer Bettemperatur von ca. 40° C. Die Ablufttemperatur beträgt ca. 35-40° C. Es ist darauf zu achten, dass die Abluft möglichst gesättigt ist (ca. 80 90% rel. Feuchte). Das Überkorn aus dem rückzuführenden Produkt (9B,10 B) wird ausgesiebt, da dieses sich sonst in der Sprühzone weiter anreichert und im Fließbett Probleme bereitet. Das ausgesiebte Unter- und Überkorn wird saugseitig dem Ventilator (E) zugeführt und gemahlen. Es wird zusammen mit den anderen rückgeführten Feststoffteilströmen (9 A, 10 A, 9 B, 10 B) wieder dem Prozess zugeführt.

### Beispiel 3

### Herstellung eines DC β-Mannits mit einem mittleren Korn X₅₀ = 450 µm

Wie in Beispiel 1 beschrieben, wird die Sprühtrocknungsanlage zur Vorbereitung mit ca. 120 kg/m² β-Mannit als Bett befüllt. Als Wirbel- und Zuluft wird die Anlage mit 1700 m³/m² h bei einer Temperatur von ca. 100° C betrieben.

Das Heißgas wird in einer Größenordnung von ca. 1,6 m³/(h kg Lösung) bei einer Temperatur von ca. 80° C der Sprühzone zugeführt. Sind alle diese Parameter eingestellt, kann mit dem Versprühen von Mannitlösung begonnen werden.

Die Lösung hat eine Mannit-Konzentration von ca. 50 Gew.-% bei einer Temperatur von ca. 90° C. Bei einem Sprühdruck von ca. 3,5 bar (Sprühmedium ist Luft.) wird eine Lösungsmenge von ca. 100 kg/m² h in der Anlage versprüht. Über die Pulverdosierung (D) wird über die Rückführung (9, 10) Bett/Produkt von ca. 0,8 - 1,0 kg Feststoff/(h kg Lösung) in die Sprühzone rückgeführt. Die Zellräder (10A, 10B) werden so geregelt, so dass immer eine ausreichende Menge Produkt (9A, 10A) im Ventilator (E) gemahlen und mit dem ungemahlenen Produkt (9B, 10 B) wieder in die Anlage gefördert wird.

In der Anlage bildet sich durch die Verdunstung des Wassers ein Gleichgewicht mit einer Bettemperatur von ca. 40-45° C. Die Ablufttemperatur beträgt ca. 40° C. Es ist darauf zu achten, dass die Abluft möglichst gesättigt ist (ca. 80 90% rel. Feuchte).

Das Überkorn aus dem rückzuführenden Produkt (9 B,10 B) wird ausgesiebt, da dieses sich sonst in der Sprühzone anreichert und im Fließbett Probleme bereitet. Das ausgesiebte Unter- und Überkorn wird saugseitig dem Ventilator (E) zugeführt und gemahlen. Es wird zusammen mit den anderen rückgeführten Feststoffteilströmen (9A, 10 A / 9B, 10 B) wieder dem Prozess zugegeben.

## Patentansprüche

1. Verfahren zur Herstellung von direkt tablettierbarem Mannit mit einem Gehalt an Mannit in β-Modifikation von mehr als 90 %, **dadurch gekennzeichnet, dass**
a) in einem ersten Schritt als Edukt eine wässrige 40 - 50%-ige D-Mannit-Lösung mit einer Temperatur im Bereich von 60 bis 95 °C mit Sprühgas, pulverförmigem β-Mannit und Heißgas zusammengeführt wird,
wobei der Sprühdruck der verwendeten Zweistoffdüse im Bereich von 2 - 4 bar eingestellt und 1,5 bis 3 m³/(h kg Lösung) Heißgas mit einer Temperatur von 80 bis 110 °C als Sprühgas der Zweistoffdüse zugeführt wird und die der Anlage zugeführte Luft (4) auf eine Temperatur im Bereich von 45 - 120 °C vorgewärmt wird und in einer Menge im Bereich von 1000 - 2000 m³/m² pro Stunde zugeführt wird, wodurch sich die Ablufttemperatur im Bereich von 30 - 50 °C einstellt,
b) das entstehende pulverförmige Produkt in ein Wirbel- oder Fließbett fällt, aufgenommen, fluidisiert und weitertransportiert wird, und gegebenenfalls in einem oder mehreren Granulierungsschritt(en) mit weiterem flüssigen Medium besprüht, getrocknet und im Fließ- oder Wirbelbett weitertransportiert wird, und
c) durch Rückführung in einer Menge im Bereich von 0,2 - 2,0 kg Feststoff/(h kg Lösung) des als Staubanteil aus der Produktaustragszone des Prozessors anfallenden β-Mannits in den Schritt a) der Sprühtrocknung das Gleichgewicht hin zur Bildung von β-Mannit verschoben wird.
wobei durch Rückführung eines Pulvers mit Teilchengrößen kleiner 20 µm Mannitpulver mit einem Anteil an β-Fraktion von mehr als 90% erhalten wird.

2. Verfahren gemäß Anspruch 1, **dadurch gekennzeichnet, dass** zur Herstellung der eingesetzten Mannit-Lösung D-Mannit mit einer Reinheit von >95 % verwendet wird.

3. Verfahren gemäß Anspruch 1, **dadurch gekennzeichnet, dass** zur Herstellung der eingesetzten Mannit-Lösung D-Mannit mit einer Reinheit von >98 % verwendet wird.

4. Verfahren gemäß Anspruch 1 **dadurch gekennzeichnet, dass** in a) β-Mannit mit einer durchschnittlichen Partikelgröße im Bereich von 1 bis 20 µm zurückgeführt wird.

5. Verfahren gemäß der Ansprüche 1 und 4 **dadurch gekennzeichnet, dass** das "staubförmige" β-Mannit, das in der Leitung (9A) anfällt, aus der Pulverdosieranlage als pulverförmiges β-Mannit durch Steuerung der Drehzahl der Zellradschleuse (10 A) über den Ventilator (E) in die Sprühtrocknung (Schritt a)) rückgeführt wird.

6. Verfahren gemäß der Ansprüche 1, 4 und 5, **dadurch gekennzeichnet, dass** nach der Einstellung des Gleichgewichts pulverförmiges β-Mannit mit einer durchschnittlichen Partikelgröße kleiner als 75 µm rückgeführt wird.

7. Verfahren gemäß der Ansprüche 1 und 4 bis 6, **dadurch gekennzeichnet, dass** nach der Einstellung des Gleichgewichts pulverförmiges unvermahlenes β-Mannit rückgeführt wird.

8. Verfahren gemäß (der) Ansprüch(e) 6 und/oder 7, **dadurch gekennzeichnet, dass** das zurückgeführte pulverförmige Material vor der Rückführung durch Vermahlen im Ventilator (E), der gleichzeitig als Förderorgan für die Pulverrückführung dient, zerkleinert wird.

9. Verfahren gemäß der Ansprüche 1 und 4 bis 8, **dadurch gekennzeichnet, dass** durch Regelung der Drehzahlen der Zellradschleusen 10A und 10B und durch Vermahlen des anfallenden grobkörnigen Produkts im Ventilator (E) auf Partikelgrößen kleiner 75 µm vor der Rückführung in die Sprühtrocknung die alleinige Bildung von β-Mannit erfolgt.

10. Verfahren gemäß Anspruch 1, **dadurch gekennzeichnet, daß** sowohl als Sprühgas als auch als Träger- und Heizgas Luft oder ein Inertgas ausgewählt aus der Gruppe N₃ und CO₂ verwendet wird.

11. Verfahren gemäß Anspruch 1, **dadurch gekennzeichnet, daß** das Gas im Kreislauf geführt wird und das im Kreislauf geführte Gas durch Filter von Partikeln befreit wird, im Kondensator getrocknet und erneut den Sprühdüsen zugeführt bzw. aufgeheizt und in das Fließbett eingeführt wird.

12. Verfahren gemäß Anspruch 11, **dadurch gekennzeichnet, dass** das Gas mit Hilfe von Dynamikfiltern von Partikeln befreit wird.

13. Verfahren gemäß Anspruch 1, **dadurch gekennzeichnet, dass** die verwendeten flüssigen Medien an verschiedenen Stellen der Anlage unterschiedliche Zusammensetzungen aufweisen.

14. Verfahren gemäß der Ansprüche 1 bis 13, **dadurch gekennzeichnet, dass** durch Variation der Parameter, Sprühdruck, Sprühmenge, Mannitkonzentration, zurückgeführte Pulvermenge, Heißluftstrom und Temperatur der Heißluft gezielt Partikelgrößen zwischen 50 bis 1000 µm hergestellt werden.

15. Verfahren gemäß Anspruch 14, **dadurch gekennzeichnet, dass** durch Einstellung der Parameter, Sprühdruck, Flüssigkeitsmenge, Mannitkonzentration, zurückgeführte Pulvermenge, Heißluftstrom und Temperatur der Heißluft die im Wirbel- oder Fließbett befindliche Pulvermenge auf eine Bettmenge im Bereich von 50 - 150 kg/m² Bett eingestellt wird.

## Claims

1. Process for the preparation of directly compressible mannitol having a mannitol content in the β modification of greater than 90%, **characterised in that**
a) in a first step, as starting material, an aqueous 40 - 50% D-mannitol solution having a temperature in the range from 60 to 95°C is combined with spray gas, pulverulent β-mannitol and hot gas,
where the spray pressure of the two-component nozzle used is set in the range 2 - 4 bar, and 1.5 to 3 m³/(h kg of solution) of hot gas having a temperature of 80 to 110°C is fed to the two-component nozzle as spray gas, and the air (4) fed to the apparatus is pre-warmed to a temperature in the range 45 - 120°C and is supplied in an amount in the range 1000 - 2000 m³/m² per hour, causing the exhaust-air temperature to become established in the range 30 - 50°C,
b) the pulverulent product formed falls into a fluidised bed, is taken up, fluidised and transported further, and optionally sprayed with further liquid medium in one or more granulation step(s), dried and transported further in the fluidised bed, and
c) the equilibrium is shifted towards the formation of β-mannitol by recycling, in an amount in the range from 0.2 - 2.0 kg of solid/(h/kg of solution), the β-mannitol formed as dust component from the product discharge zone of the processor into step a) of the spray drying,
where, by recycling of a powder having particle sizes smaller than 20 µm, mannitol powder having a proportion of β particles of greater than 90% is obtained.

2. Process according to Claim 1, **characterised in that**, for the preparation of the mannitol solution employed, use is made of D-mannitol having a purity of >95%.

3. Process according to Claim 1, **characterised in that**, for the preparation of the mannitol solution employed, use is made of D-mannitol having a purity of >98%.

4. Process according to Claim 1, **characterised in that** β-mannitol having a mean particle size in the range from 1 to 20 µm is recycled into a).

5. Process according to Claims 1 and 4, **characterised in that** the "dust-form" β-mannitol formed in line (9A) is recycled into the spray drying (step a)) from the powder-metering device as pulverulent β-mannitol by controlling the rotational speed of the star valve (10A) via the fan (E).

6. Process according to Claims 1, 4 and 5, **characterised in that**, after the equilibrium has been established, pulverulent β-mannitol having a mean particle size of less than 75 µm is recycled.

7. Process according to Claims 1 and 4 to 6, **characterised in that**, after the equilibrium has been established, pulverulent unground β-mannitol is recycled.

8. Process according to Claim(s) 6 and/or 7, **characterised in that** the recycled pulverulent material is comminuted, before the recycling, by grinding in the fan (E), which simultaneously serves as conveying element for the powder recycling.

9. Process according to Claims 1 and 4 to 8, **characterised in that** regulation of the rotational speeds of the star valves 10A and 10B and grinding of the coarse product formed to particle sizes of less than 75 µm in the fan (E) before recycling into the spray drying result in the exclusive formation of β-mannitol.

10. Process according to Claim 1, **characterised in that** air or an inert gas selected from the group of N₂ and CO₂ is used both as spray gas and also as carrier and heating gas.

11. Process according to Claim 1, **characterised in that** the gas is circulated, and the circulated gas is freed from particles by filters, dried in the condenser and fed back to the spray nozzles or heated and introduced into the fluidised bed.

12. Process according to Claim 11, **characterised in that** the gas is freed from particles with the aid of dynamic filters.

13. Process according to Claim 1, **characterised in that** the liquid media used have different compositions at different points of the plant.

14. Process according to Claims 1 to 13, **characterised in that** particle sizes of between 50 and 1000 µm are produced specifically by varying the parameters spray pressure, spray amount, mannitol concentration, amount of powder recycled, hot-air stream and hot-air temperature.

15. Process according to Claim 14, **characterised in that** the amount of powder present in the fluidised bed is set to an amount in the range 50 - 150 kg/m² of bed by adjustment of the parameters spray pressure, amount of liquid, mannitol concentration, amount of powder recycled, hot-air stream and hot-air temperature.

## Revendications

1. Procédé de production de mannitol directement façonnable en comprimés, ayant une teneur de mannitol en modification β de plus de 90%, **caractérisé en ce que**
a) au niveau d'une première étape, en tant que matériau de départ, une solution de D-mannitol aqueuse à 40 - 50% présentant une température dans la plage de 60 à 95°C est combinée avec un gaz de pulvérisation, du β-mannitol pulvérulent et un gaz chaud, où la pression de pulvérisation de la buse à deux composants qui est utilisée est établie dans la plage de 2 - 4 bars, et 1,5 à 3 m³/(h kg de solution) de gaz chaud présentant une température de 80 à 110°C sont alimentés sur la buse à deux composants en tant que gaz de pulvérisation, et de l'air (4) qui est alimenté sur l'appareil est préchauffé jusqu'à une température dans la plage de 45 - 120°C et est appliqué selon une quantité dans la plage de 1000 - 2000 m³/m² par heure, ce qui a pour effet que la température de l'air d'évacuation en vient à être établie dans la plage de 30 - 50°C,
b) le produit pulvérulent formé est précipité selon un lit fluidisé, est repris, est fluidisé et est transporté plus loin et en option, est pulvérisé avec un autre milieu liquide au niveau d'une ou de plusieurs étapes de granulation, est séché et est transporté plus loin dans le lit fluidisé ; et
c) l'équilibre est décalé en direction de la formation de β-mannitol par recyclage, selon une quantité dans la plage de 0,2 - 2,0 kg de solide (h/kg de solution), du β-mannitol formé en tant que composant de poussière en provenance de la zone de décharge de produit du processeur au niveau de l'étape a) du séchage par pulvérisation,
dans lequel, par recyclage d'une poudre ayant des dimensions de particules inférieures à 20 µm, une poudre de mannitol ayant une proportion de particules β supérieure à 90% est obtenue.

2. Procédé selon la revendication 1, **caractérisé en ce que** l'on utilise, pour préparer la solution de mannitol mise en oeuvre, du D-mannitol d'une pureté > 95%.

3. Procédé selon la revendication 1, **caractérisé en ce que** l'on utilise, pour préparer la solution de mannitol mise en oeuvre, du D-mannitol d'une pureté > 98%.

4. Procédé selon la revendication 1, **caractérisé en ce que** du β**-**mannitol présentant une dimension moyenne de particules dans la plage de 1 à 20 µm est recyclé au niveau de a).

5. Procédé selon les revendications 1 et 4, **caractérisé en ce que** l'on recycle le β-mannitol "en poussière", qui est produit dans la conduite (9A), dans l'étape de séchage par pulvérisation (étape a)) sous forme de β-mannitol pulvérulent à partir du doseur de poudre, par l'intermédiaire du ventilateur (E) en régulant la vitesse de rotation du déversoir à roue à godets (10A).

6. Procédé selon les revendications 1, 4 et 5, **caractérisé en ce qu'**après établissement de l'équilibre, on recycle le β-mannitol pulvérulent avec une granulométrie moyenne inférieure à 75 µm.

7. Procédé selon les revendications 1 et 4 à 6, **caractérisé en ce qu'**après établissement de l'équilibre, on recycle le β-mannitol pulvérulent non broyé.

8. Procédé selon la (les) revendication(s) 6 et/ou 7, **caractérisé en ce que** l'on broie la matière pulvérulente recyclée, avant le recyclage, par broyage dans le ventilateur (E), qui sert également d'organe de transport pour le recyclage de la poudre.

9. Procédé selon les revendications 1 et 4 à 8, **caractérisé en ce que** l'on réalise la formation exclusive du β-mannitol en régulant la vitesse de rotation des déversoirs à roue à godets 10A et 10B et en broyant le produit granuleux grossier résultant dans le ventilateur (E) à une granulométrie inférieure à 75 µm avant le recyclage dans le séchage par pulvérisation.

10. Procédé selon la revendication 1, **caractérisé en ce que** de l'air ou un gaz inerte choisi parmi le groupe comprenant N₂ et CO₂ est utilisé à la fois en tant que gaz de pulvérisation et également en tant que gaz porteur et chauffant.

11. Procédé selon la revendication 1, **caractérisé en ce que** l'on fait circuler le gaz en boucle et **en ce que** le gaz circulant en boucle est libéré des particules au moyen de filtres, séché dans le condenseur et reconduit dans les buses de pulvérisation ou chauffé et introduit dans le lit fluidisé.

12. Procédé selon la revendication 11, **caractérisé en ce que** le gaz est libéré des particules à l'aide de filtres dynamiques.

13. Procédé selon la revendication 1, **caractérisé en ce que** les milieux liquides utilisés présentent différentes compositions en différents points de l'installation.

14. Procédé selon les revendications 1 à 13, **caractérisé en ce que** l'on obtient de manière ciblée des granulométries comprises entre 50 et 1000 µm en faisant varier les paramètres pression de pulvérisation, quantité pulvérisée, concentration de mannitol, quantité de poudre recyclée, courant d'air chaud et température de l'air chaud.

15. Procédé selon la revendication 14, **caractérisé en ce que** la quantité de poudre se trouvant dans le lit (fluide ou) fluidisé est ajustée à une quantité de lit allant de 50 à 150 kg/m² de lit en ajustant les paramètres pression de pulvérisation, quantité de liquide, concentration de mannitol, quantité de poudre recyclée, courant d'air chaud et température de l'air chaud.
